# EUROPEAN PATENT APPLICATION

(11) **EP 3 903 773 A1**
(43) Date of publication of application: **03.11.2021**
(21) Application number: 19904673.1
(22) Date of filing: 23.12.2019
(51) Int. Cl.: A61K 31/12, A61P 39/06, C07C 45/79, C07C 49/84, A61K 36/185, A23L 33/105

(54) **METHOD FOR PRODUCING XANTHOHUMOL-CONTAINING COMPOSITION**

(30) Priority: 27.12.2018 JP 2018245317; 09.07.2019 JP 2019127608
(71) Applicant: Suntory Holdings Limited, Osaka 530-8203 (JP)
(72) Inventor: HATA, Yuto, Soraku-gun, Kyoto 619-0284 (JP); YOSHII, Takaaki, Soraku-gun, Kyoto 619-0284 (JP); TAKAGI, Risa, Soraku-gun, Kyoto 619-0284 (JP); NAKAHARA, Koichi, Soraku-gun, Kyoto 619-0284 (JP)
(74) Representative: Vossius & Partner Patentanwälte Rechtsanwälte mbB
(86) International application number: PCT/JP2019/050366
(87) International publication number: WO 2020/137975

(57) **Abstract**

The present invention relates to, for example, a method of producing a xanthohumol-containing composition, including: contacting a first mixture of a hop-derived, xanthohumol-containing component and a first solvent containing water and a water-miscible solvent with polyvinylpolypyrrolidone to adsorb xanthohumol on the polyvinylpolypyrrolidone; washing the polyvinylpolypyrrolidone containing xanthohumol adsorbed thereon with a second solvent; and eluting xanthohumol from the polyvinylpolypyrrolidone with a third solvent to obtain a xanthohumol-containing eluate, wherein the second solvent is an aqueous ethanol solution, and the third solvent is ethanol or an aqueous ethanol solution having a higher concentration than the second solvent.

## Description

### TECHNICAL FIELD

The present invention relates to a method of producing a xanthohumol-containing composition.

### BACKGROUND ART

Xanthohumol is a compound contained in plants called hops (scientific name: *Humulus lupulus,* Cannabaceae) used as raw materials of beer. Xanthohumol is a type of polyphenol. Xanthohumol has been reported to have physiological effects such as antioxidant effect. Compositions such as hop extract containing xanthohumol, particularly, compositions having a high xanthohumol content, are useful in the heath food field and the like.

Non-Patent Literature 1 describes a method of obtaining a xanthohumol-containing powder by passing hop extract through polyvinylpolypyrrolidone to adsorb prenylflavonoids thereon, eluting prenylflavonoids with ethyl acetate, and evaporating the solvent from the eluate.

### CITATION LIST

### - Non-Patent Literature

Non-Patent Literature 1: M. Biendl., "COMMERCIAL HOP EXTRACTS RICH IN XANTHOHUMOL", ISSN 1814-2206 International Hop Growers" Convention I.H.G.C., Proceedings of the Scientific Commission, 2007, pp. 41-44

### SUMMARY OF INVENTION

### - Technical Problem

According to Non-Patent Literature 1, when the xanthohumol-containing powder (xanthohumol content: 65 to 85%) obtained by separation using polyvinylpolypyrrolidone is further subjected to a recrystallization step, it is possible to further increase the xanthohumol content. Yet, the method will involve many operations when the recrystallization step is performed, which complicates the production. In Non-Patent Literature 1, ethyl acetate is used when xanthohumol is eluted from polyvinylpolypyrrolidone, however, in Japan, for example, the intended use of ethyl acetate is limited when used in food and beverages. Since xanthohumol has useful physiological effects as described above, a method capable of efficiently producing a xanthohumol-containing composition that can be widely used in food and beverage production and the like is useful.

The present invention aims to provide a novel method of efficiently producing a xanthohumol-containing composition that can be widely used in food and beverage production and the like.

### - Solution to Problem

As a result of extensive studies, the present inventors found that when a mixture of a hop-derived, xanthohumol-containing component and a solvent containing water and a water-miscible solvent is contacted with polyvinylpolypyrrolidone (hereinafter also referred to as "PVPP") to adsorb xanthohumol thereon, followed by washing with an aqueous ethanol solution and then elution of xanthohumol with a solvent having a higher ethanol concentration than the aqueous ethanol solution, it is possible to suitably separate xanthohumol from other hop-derived component(s), which enables efficient production of a composition having a high xanthohumol content in solids. The present inventors also found that when a mixture of a hop-derived, xanthohumol-containing component and an aqueous ethanol solution having a specific concentration is contacted with PVPP to adsorb xanthohumol thereon, followed by elution of xanthohumol with a solvent having a higher ethanol concentration than the aqueous ethanol solution, it is also possible to suitably separate xanthohumol from other hop-derived component(s). Ethanol can be widely used in food and beverages, and a xanthohumol-containing composition obtained with ethanol for elution from PVPP can be widely used in food and beverage production and the like.

Specifically, the present invention relates to the following methods of producing a xanthohumol-containing composition.
(1) A method of producing a xanthohumol-containing composition, including: contacting a first mixture of a hop-derived, xanthohumol-containing component and a first solvent containing water and a water-miscible solvent with polyvinylpolypyrrolidone to adsorb xanthohumol on the polyvinylpolypyrrolidone; washing the polyvinylpolypyrrolidone containing xanthohumol adsorbed thereon with a second solvent; and eluting xanthohumol from the polyvinylpolypyrrolidone with a third solvent to obtain a xanthohumol-containing eluate, wherein the second solvent is an aqueous ethanol solution, and the third solvent is ethanol or an aqueous ethanol solution having a higher concentration than the second solvent.
(2) The production method according to (1) above, wherein the second solvent is a 30 to 70 vol% aqueous ethanol solution.
(3) The production method according to (1) or (2) above, wherein the third solvent is a 70 or more vol% aqueous ethanol solution.
(4) The production method according to any one of (1) to (3) above, wherein the first solvent contains 30 to 80 vol% water and a 20 to 70 vol% water-miscible solvent.
(5) The production method according to any one of (1) to (4) above, wherein the water-miscible solvent is ethanol.
(6) A method of producing a xanthohumol-containing composition, including: contacting a third mixture of a hop-derived, xanthohumol-containing component and a fifth solvent with polyvinylpolypyrrolidone to adsorb xanthohumol on the polyvinylpolypyrrolidone; and eluting xanthohumol from the polyvinylpolypyrrolidone containing xanthohumol adsorbed thereon with a sixth solvent to obtain a xanthohumol-containing eluate, wherein the fifth solvent is a 50 to 70 vol% aqueous ethanol solution, and the sixth solvent is ethanol or an aqueous ethanol solution having a higher concentration than the fifth solvent.
(7) The production method according to (6) above, wherein the sixth solvent is a 70 or more vol% aqueous ethanol solution.
(8) The production method according to any one of (1) to (7) above, further including a step of removing the solvent from the xanthohumol-containing eluate.
(9) The production method according to any one of (1) to (8) above, which is a method of producing a composition having a xanthohumol content of 80 to 99 wt%.

### - Advantageous Effects of Invention

The present invention can provide a novel method of producing a xanthohumol-containing composition. The production method of the present invention can efficiently produce a xanthohumol-containing composition. The xanthohumol-containing composition obtained by the production method of the present invention can be widely used in food and beverage production and the like.

### DESCRIPTION OF EMBODIMENTS

The method of producing a xanthohumol-containing composition according to a first embodiment of the present invention (hereinafter also referred to as a "production method according to the first embodiment") includes a step of contacting a first mixture of a hop-derived, xanthohumol-containing component and a first solvent containing water and a water-miscible solvent with polyvinylpolypyrrolidone to adsorb xanthohumol on the polyvinylpolypyrrolidone (also referred to as an "adsorption step"); a step of washing the polyvinylpolypyrrolidone containing xanthohumol adsorbed thereon with a second solvent (also referred to as a "washing step"); and a step of eluting xanthohumol from the polyvinylpolypyrrolidone with a third solvent to obtain a xanthohumol-containing eluate (also referred to as an "elution step"). In the production method according to the first embodiment of the present invention, the second solvent is an aqueous ethanol solution, and the third solvent is ethanol or an aqueous ethanol solution having a higher concentration than the second solvent.

A method of producing a xanthohumol-containing composition according to a second embodiment of the present invention (hereinafter also referred to as a "production method according to the second embodiment") includes contacting a third mixture of a hop-derived, xanthohumol-containing component and a fifth solvent with polyvinylpolypyrrolidone to adsorb xanthohumol on the polyvinylpolypyrrolidone (also referred to as an "adsorption step"); and a step of eluting xanthohumol from the polyvinylpolypyrrolidone containing xanthohumol adsorbed thereon with a sixth solvent to obtain a xanthohumol-containing eluate (also referred to as an "elution step"). In the method of producing a xanthohumol-containing composition according to the second embodiment of the present invention, the fifth solvent is a 50 to 70 vol% (v/v%) aqueous ethanol solution, and the sixth solvent is ethanol or an aqueous ethanol solution having a higher concentration than the fifth solvent.

First, the production method according to the first embodiment of the present invention is described.

The production method according to the first embodiment of the present invention includes the adsorption step, the washing step, and the elution step. The production method according to the first embodiment may include other step(s) as long as the effect of the present invention is not impaired. For example, the production method may include a step of preparing the first mixture (also referred to as a "first mixture preparation step"). Usually, the first mixture is prepared before the adsorption step. The production method according to the first embodiment of the present invention may include a step of removing the solvent from the xanthohumol-containing eluate obtained in the elution step (also referred to as a "solvent removal step").

The first mixture is a mixture of a hop-derived, xanthohumol-containing component and a first solvent containing water and a water-miscible solvent. The hop-derived component can also be referred to as a hop-derived substance. The first mixture may be a solution in which a hop-derived, xanthohumol-containing component is dissolved in the first solvent, or may be a suspension of the hop-derived component.

The solvent containing water and a water-miscible solvent may be a solvent consisting of water and a water-miscible solvent. Usually, the hop-derived component contained in the first mixture and a third mixture (described later) contains a component other than xanthohumol.

Examples of the water-miscible solvent include C1-C4 monohydric alcohols such as methanol, ethanol, n-propanol, isopropanol, n-butanol, s-butanol, isobutanol, and t-butanol; C2-C4 diols such as ethylene glycol, propylene glycol, and butylene glycol; ketones such as acetone; cyclic ethers such as tetrahydrofuran; amide-based solvents such as dimethylformamide and dimethylacetamide; amine-based solvents such as dimethylamine; sulfoxide-based solvents such as dimethyl sulfoxide; nitrile-based solvents such as acetonitrile; and organic acid solvents such as acetic acid. These water-miscible solvents may be used alone or in combination of two or more thereof. Of these, C1-C4 monohydric alcohols and C2-C4 diols are preferred, and ethanol is more preferred. Ethanol is preferred because it can be widely used in food and beverages. In addition, the presence of ethanol in the first mixture advantageously improves adsorption of xanthohumol on PVPP.

In order to enhance adsorption of xanthohumol on PVPP, preferably, the first solvent contains 30 to 80 vol% water and a 20 to 70 vol% water-miscible solvent. The water concentration and the water-miscible solvent concentration in the first solvent are more preferably 35 to 75 vol% and 25 to 65 vol%, respectively, still more preferably 40 to 70 vol% and 30 to 60 vol%, respectively. The first solvent is preferably an aqueous ethanol solution, and the ethanol concentration in the aqueous solution is more preferably 20 to 70 vol%, more preferably 20 to 65 vol%, still more preferably 25 to 60 vol%, particularly preferably 30 to 60 vol%.

In the production method according to the first embodiment of the present invention, the first mixture may be prepared and contacted with polyvinylpolypyrrolidone. The first mixture may be one that was provided, or may be prepared by the step of preparing the first mixture.

The first mixture may be prepared by any method. For example, a hop-derived, xanthohumol-containing component can be extracted from a hop-derived, xanthohumol-containing raw material with a solvent. More specifically, the extraction results in a mixture of a hop-derived, xanthohumol-containing component and a solvent.

Examples of the hop-derived, xanthohumol-containing raw material include hop cones, residue of hops (preferably, hop cones) extracted with supercritical carbon dioxide (also referred to as "spent hops"), residue of spent hops extracted with water, dried hop cones, pulverized dried hop (preferably, hop cones). Of these, residue of hopes extracted with supercritical carbon dioxide is preferred.

In one embodiment, the first mixture preparation step may include extracting a hop-derived, xanthohumol-containing raw material with a fourth solvent containing a water-miscible solvent. In one embodiment, the first mixture can be prepared by, for example, extracting a hop-derived, xanthohumol-containing raw material with the fourth solvent containing a water-miscible solvent to prepare a second mixture containing a hop-derived, xanthohumol-containing component, and optionally adjusting the water concentration and the water-miscible solvent concentration in the second mixture. Usually, the second mixture is a liquid extract obtained by extracting the hop-derived raw material with the fourth solvent. In one embodiment, the second mixture can be used directly as the first mixture. In a preferred embodiment, the water concentration and the water-miscible solvent concentration in the second mixture are adjusted to the preferred ranges in the first mixture described above.

In one embodiment of the present invention, when a hop-derived, xanthohumol-containing raw material is extracted with a solvent, preferably, the raw material is washed with a solvent containing water before extraction. When the hop-derived raw material is washed with a solvent containing water before extraction, the resulting xanthohumol-containing composition has a higher xanthohumol content in solids. In one embodiment, preferably, the hop-derived, xanthohumol-containing raw material is washed with a solvent containing water, and the hop-derived, xanthohumol-containing raw material is then extracted with the fourth solvent containing a water-miscible solvent to prepare a liquid extract containing a hop-derived, xanthohumol-containing component (the second mixture) so as to prepare the first mixture.

In washing before extraction, the hop-derived raw material may be contacted with a solvent containing water. The solvent used for washing the hop-derived raw material (also referred to as a "raw material washing solvent") is a solvent containing water, and may contain a water-miscible solvent. Preferably, the raw material washing solvent is water or a mixture of water and a water-miscible solvent. The water-miscible solvent and a preferred embodiment thereof are as described above. When the raw material washing solvent contains a water-miscible solvent, preferably, the water-miscible solvent concentration is lower than the water-miscible solvent concentration in the fourth solvent used for extraction. In one embodiment, the water-miscible solvent concentration in the raw material washing solvent is preferably 30 vol% or less. When the water-miscible solvent concentration in the raw material washing solvent is 30 vol% or less, the resulting xanthohumol-containing composition has a higher xanthohumol content in solids. In order to prevent the loss of xanthohumol from the raw material, the water-miscible solvent concentration in the raw material washing solvent is preferably 30 vol% or less. The method of contacting the raw material washing solvent with the hop-derived raw material is not limited. Examples of the method include one in which the solvent and the hop-derived raw material are placed in a container and optionally stirred. The contact time between the raw material washing solvent and the hop-derived raw material is preferably 0.5 to 3 hours, more preferably 0.5 to 2 hours. The amount (in weight) of the raw material washing solvent is preferably not less than 2 times, more preferably 10 to 20 times that of the hop-derived raw material (in terms of dry matter). The temperature of the raw material washing solvent is preferably 50°C or lower when the raw material washing solvent is water, and is preferably 40°C or lower when the raw material washing solvent contains a water-miscible solvent, in order to prevent the loss of xanthohumol and conversion into isoxanthohumol. The temperature of the raw material washing solvent is more preferably 20°C to 30°C. After washing, the hop-derived raw material may be separated from the solvent and subjected to extraction with the fourth solvent. A known method may be used to separate the hop-derived raw material from the solvent. For example, when the hop-derived raw material is contacted with the raw material washing solvent in a container, the raw material can be separated from the solvent by a method such as filtration or centrifugation.

The fourth solvent used for extraction is preferably a water-miscible solvent or a mixture of water and a water-miscible solvent. A mixture of water and a water-miscible solvent is more preferred. In order to efficiently extract xanthohumol from the hop-derived raw material, preferably, the fourth solvent contains 10 to 65 vol% water and a 35 to 90 vol% water-miscible solvent; more preferably, the fourth solvent contains 30 to 60 vol% water and a 40 to 70 vol% water-miscible solvent; and still more preferably, the fourth solvent contains 30 to 50 vol% water and a 50 to 70 vol% water-miscible solvent. The water-miscible solvent and a preferred embodiment thereof are as described above, and ethanol is preferred. In one embodiment, the fourth solvent is an aqueous ethanol solution having an ethanol concentration of preferably 35 to 90 vol%, more preferably 40 to 70 vol%, still more preferably 50 to 70 vol%.

The extraction can be performed by a method used for plant extraction, and may be performed by immersing a hop-derived raw material in the fourth solvent. Here, an operation such as stirring may be suitably performed. The amount (in weight) of the fourth solvent is preferably not less than 2 times, more preferably 10 to 20 times that of the hop-derived raw material (in terms of dry matter). Preferably, the extraction is performed at 4°C to 30°C. The extraction time is not limited as long as xanthohumol is extracted, but it is preferably 30 minutes to 5 hours, more preferably 45 minutes to 2 hours, to obtain a xanthohumol-containing liquid extract.

Preferably, the extraction is followed by removal of insoluble, hop-derived raw material residue from the liquid extract. The method of removing insoluble residue from the liquid extract is not limited, and a known separation means such as filtration or centrifugation can be used. The thus-obtained liquid extract of the hop-derived raw material is collected as the second mixture containing a hop-derived, xanthohumol-containing component.

When the second mixture obtained above contains water and a water-miscible solvent, the mixture may be used as the first mixture. In one embodiment, preferably, the water concentration and the water-miscible solvent concentration in the second mixture are adjusted to the preferred ranges in the first mixture described above to prepare the first mixture. The water concentration and the water-miscible solvent concentration can be adjusted, for example, by mixing the second mixture with water or a water-miscible solvent. In one embodiment, the solvent may be partially or entirely removed from the second mixture to prepare the first mixture.

In an example of a preferred embodiment, the hop-derived, xanthohumol-containing raw material is extracted with a 35 to 90 vol% (preferably 40 to 70 vol%, more preferably 50 to 70 vol%) aqueous ethanol solution to obtain a liquid extract as the second mixture containing a hop-derived, xanthohumol-containing component, and the ethanol concentration in the second mixture is optionally adjusted to 20 to 70 vol%, whereby the first mixture can be prepared.

In one embodiment, preferably, the hop-derived, xanthohumol-containing raw material is washed with a solvent containing water (preferably water or a mixture of water and a water-miscible solvent having a water-miscible solvent concentration of 30 vol% or less), and the hop-derived, xanthohumol-containing raw material is then extracted with a 35 to 90 vol% aqueous ethanol solution to obtain a xanthohumol-containing liquid extract. The method including washing the hop-derived, xanthohumol-containing raw material and then extracting the raw material is a preferred method of preparing a liquid extract of a hop-derived, xanthohumol-containing raw material.

Alternatively, the first mixture can be prepared by dissolving or suspending a hop-derived, xanthohumol-containing component in the first solvent. For example, a xanthohumol-containing hop extract, a treated product obtained by further treating the xanthohumol-containing hop extract, or the like is mixed with a solvent, whereby the hop-derived component can be dissolved or suspended in the solvent. In the present invention, the xanthohumol-containing hop extract, its treated product, or the like may be mixed with water and a water-miscible solvent to prepare the first mixture. Examples of the hop extract, its treated product, or the like include a concentrate or dried product of a liquid extract obtained by extracting the hop-derived raw material with a solvent (e.g., a water-miscible solvent or a mixture of water and a water-miscible solvent) and residue of the concentrate or dried product extracted with supercritical carbon dioxide. Extraction conditions and the like may be suitably adjusted. For example, the above-described conditions can be employed for extraction with a solvent. In one embodiment, a concentrate or dried product of the liquid extract of the hop-derived raw material obtained by partially or entirely removing the solvent from the second mixture is mixed with water and/or a water-miscible solvent, whereby the first mixture can be prepared. The first mixture can be also prepared using commercially available hop extract. The first mixture can also be prepared by using a xanthohumol-containing eluate obtained by the elution step (described later) and optionally adjusting the water concentration and the ethanol concentration. A xanthohumol-containing composition obtained by removing the solvent from the eluate can also be used as a hop-derived, xanthohumol-containing component. It is possible to further increase the xanthohumol content by performing the present invention by using a xanthohumol-containing composition obtained by the production method of the present invention.

The hop-derived component content in the first mixture is not limited. For example, the content may be 3 wt% or less in the mixture. The first mixture may contain a substance other than the hop-derived component and the first solvent, as long as the effect of the present invention is not impaired.

In the production method according to the first embodiment and the production method according to the second embodiment of the present invention, the xanthohumol content in the hop-derived component is not limited. For example, it may be 0.4 to 90 wt%.

In the production method according to the first embodiment, in the adsorption step, the first mixture is contacted with polyvinylpolypyrrolidone to adsorb xanthohumol in the first mixture on polyvinylpolypyrrolidone.

Polyvinylpolypyrrolidone (PVPP) is a polymer of N-vinyl-2-pyrrolidone. Polyvinylpolypyrrolidone can be a commercially available product such as Divergan (trade name, available from BASF) or Polychlal (trade name, available from ISP).

In terms of adsorption efficiency of xanthohumol, the amount (in weight) of PVPP is preferably 5 to 150 times, more preferably 5 to 75 times, still more preferably 6 to 50 times, particularly preferably 7 to 25 times the amount of xanthohumol in the first mixture.

The method of contacting the first mixture with PVPP is not limited. Examples of the method include one in which the mixture and PVPP are placed in a container and stirred, and one in which the mixture is passed through a column packed with PVPP. The contact time between the first mixture and PVPP is preferably 0.5 hours or more. The contact time between the first mixture and PVPP is preferably 0.5 to 3 hours, more preferably 0.5 to 2 hours, when the mixture and PVPP are placed in a container and stirred, for example.

After the first mixture is contacted with PVPP, preferably, PVPP containing xanthohumol adsorbed thereon is separated from a solvent (a mixture of water and a water-miscible solvent). A known method may be used to separate PVPP. For example, in the case where PVPP and the first mixture are placed in a container and stirred, PVPP can be separated from the solvent by a method such as filtration or centrifugation. When the adsorption step is performed with PVPP packed in a column, the solvent in the first mixture is passed through PVPP in the column, whereby the adsorption of the component and separation of PVPP from the solvent can be performed.

In the production method according to the first embodiment, the polyvinylpolypyrrolidone containing xanthohumol adsorbed thereon is washed with the second solvent (washing step). The second solvent is an aqueous ethanol solution. Then, xanthohumol is eluted from the washed polyvinylpolypyrrolidone with ethanol or an aqueous ethanol solution (a third solvent) having a concentration higher than that of the second solvent, whereby a xanthohumol-containing eluate can be obtained (elution step). In the production method according to the first embodiment of the present invention, after PVPP containing xanthohumol adsorbed thereon is washed with the second solvent, elution is performed using the third solvent having a higher ethanol concentration than the aqueous ethanol solution used for washing. This can suitably separate xanthohumol from other component(s) in the hop-derived component and provide an eluate having a higher xanthohumol content in solids.

The washing can be performed by contacting PVPP that underwent the adsorption step with the second solvent. In one embodiment, when the first mixture used in the adsorption step contains ethanol, the ethanol concentration in the second solvent is preferably higher than or equal to the ethanol concentration in the first solvent, and is more preferably higher than the ethanol concentration in the first solvent. In order to obtain a composition having a high xanthohumol content, the second solvent is an aqueous ethanol solution having an ethanol concentration of preferably 30 to 70 vol%, more preferably 40 to 60 vol%. The washing with an aqueous ethanol solution having an ethanol concentration in the above ranges can cause elution of a hop-derived component(s) other than xanthohumol from PVPP. Thus, xanthohumol can be suitably separated from other component(s). The ethanol concentration in the second solvent is more preferably 50 to 60 vol%. The ethanol concentration in the above range improves the yield of xanthohumol.

The amount (in volume) of the second solvent in the washing step is preferably not less than 2 times, more preferably not less than 3 times that of PVPP. In order to reduce the ethanol cost and to prevent the loss of the target component, the amount (in volume) of the second solvent is preferably not more than 40 times, more preferably not more than 30 times, still more preferably not more than 20 times, particularly preferably not more than 10 times that of PVPP. In one embodiment, the amount (in volume) of the second solvent is 2 to 40 times, more preferably 2 to 30 times, still more preferably 2 to 20 times, particularly preferably 3 to 10 times that of PVPP. The washing with the second solvent may be performed multiple times. In that case, preferably, the total amount of the second solvent is the amount described above.

In the washing step and the elution step, the method of contacting PVPP with the second solvent or third solvent is not limited. Examples of the method include one in which the solvent and PVPP are placed in a container and stirred, and one in which the solvent is passed through a column packed with PVPP.

In the washing step, the contact time between PVPP and the second solvent is preferably 0.5 hours or more. The contact time between PVPP and the second solvent is preferably 0.5 to 3 hours, more preferably 0.5 to 2 hours, when the solvent and PVPP are placed in a container and stirred, for example.

After washing with the second solvent, PVPP is separated from the solvent and subjected to elution with the third solvent. A known method may be used to separate PVPP from the solvent. For example, when PVPP and the second solvent were placed in a container and stirred, PVPP can be separated from the solvent by a method such as filtration or centrifugation. When the washing step is performed with PVPP packed in a column, the second solvent is passed through the column packed with PVPP, whereby the washing and separation of PVPP from the solvent can be performed.

In the elution step, xanthohumol is eluted from the washed polyvinylpolypyrrolidone with the third solvent to obtain a xanthohumol-containing eluate. The third solvent is ethanol or an aqueous ethanol solution having a higher ethanol concentration than the second solvent. The elution step can be performed by contacting the above-described PVPP with the third solvent.

The third solvent is preferably ethanol or a 70 or more vol% aqueous ethanol solution, more preferably a 70 or more vol% aqueous ethanol solution, still more preferably a 70 to 99 vol% aqueous ethanol solution. In order to improve the yield of xanthohumol, the ethanol concentration in the aqueous ethanol solution used as the third solvent is more preferably 70 to 95 vol%, particularly preferably 70 to 92 vol%, most preferably 70 to 90 vol%.

The amount (in volume) of the third solvent is preferably not less than 5 times, more preferably not less than 10 times that of PVPP, in order to sufficiently eluate xanthohumol. When the amount of the third solvent used for elution is large, removal of the solvent from the resulting eluate may take time. An increase in the amount of ethanol increases the cost. In view of the above, the amount (in volume) of the third solvent is preferably not more than 100 times, more preferably not more than 70 times, still more preferably not more than 50 times, yet still more preferably not more than 40 times, particularly preferably not more than 30 times that of PVPP. In one embodiment, the amount (in volume) of the third solvent is preferably 5 to 100 times, more preferably 5 to 70 times, still more preferably 5 to 50 times, yet still more preferably 10 to 40 times, particularly preferably 10 to 30 times that of PVPP. The elution with the third solvent may be performed multiple times. When the elution is performed multiple times, preferably, the total amount of the third solvent used for elution is in the above ranges.

In the elution step, the contact time between PVPP and the third solvent is preferably 0.5 hours or more. The contact time between PVPP and the third solvent is preferably 0.5 to 3 hours, more preferably 0.5 to 2 hours, when the solvent and PVPP are placed in a container and stirred, for example.

After PVPP is contacted with the third solvent to elute xanthohumol, usually PVPP is separated from the eluate. The method of separating the eluate from PVPP is not limited, and any of the known methods described above can be used. For example, when PVPP and the third solvent were placed in a container and stirred, PVPP can be separated from the resulting eluate by a method such as filtration or centrifugation. When the elution step is performed with PVPP packed in a column, the third solvent is passed through PVPP in the column, whereby the elution of xanthohumol and separation of PVPP from the eluate can be performed.

Described below is an embodiment of the method of obtaining a xanthohumol-containing eluate by the adsorption step, the washing step, and the elution step, in the production method according to the first embodiment of the present invention.

For example, the first mixture and PVPP are placed in a container and stirred, whereby xanthohumol in the mixture can be adsorbed on PVPP. Then, PVPP containing xanthohumol adsorbed thereon is separated from the solvent. The collected PVPP and the second solvent used for washing are placed in a container and stirred. Then, PVPP is separated from the solvent. The collected PVPP and the third solvent are placed in a container and stirred, whereby xanthohumol adsorbed on PVPP is eluted. PVPP is separated from the eluate, whereby a xanthohumol-containing eluate can be obtained.

In the above embodiment, after adsorption on PVPP and washing, a column is packed with the collected PVPP and the third solvent is passed through the column so as to elute xanthohumol from PVPP and separate and obtain an eluate from PVPP.

In another embodiment to obtain an eluate, a column is packed with PVPP, the first mixture is passed through the column to adsorb xanthohumol on PVPP, and subsequently, the second solvent is passed through the column for washing. Then, the third solvent is passed through the column, whereby a xanthohumol-containing eluate can be obtained.

Next, a method of producing a xanthohumol-containing composition according to the second embodiment of the present invention is described. The production method according to the second embodiment of the present invention includes the adsorption step and the elution step. In the method of producing a xanthohumol-containing composition according to the second embodiment of the present invention, the fifth solvent is an aqueous ethanol solution having an ethanol concentration of 50 to 70 vol%, and the sixth solvent is ethanol or an aqueous ethanol solution having a higher concentration than that of the fifth solvent.

The production method according to the second embodiment of the present invention may include other step(s) as long as the effect of the present invention is not impaired. For example, the production method may include a step of preparing the third mixture (also referred to as a "third mixture preparation step"). Usually, the third mixture is prepared before the adsorption step. The production method according to the second embodiment of the present invention may include a step of removing the solvent from the xanthohumol-containing eluate obtained in the elution step (also referred to as a "solvent removal step").

The production method according to the second embodiment may or may not include a step of washing PVPP containing xanthohumol adsorbed thereon with a solvent containing water (also referred to as a "washing step") after the adsorption step and before the elution step. In one embodiment, preferably, the production method according to the second embodiment does not include the washing step of washing PVPP.

In the production method according to the second embodiment, the fifth solvent having an ethanol concentration in the above range is used for adsorption of xanthohumol on PVPP, and elution is then performed with the solvent having a higher ethanol concentration than the fifth solvent. This enables suitable separation of xanthohumol from other component(s) in the hop-derived component, without performing the washing step before the elution step. Thus, it is possible to obtain an eluate having a high xanthohumol content in solids.

When the production method according to the second embodiment includes the washing step, a solvent used for washing (i.e. an eighth solvent) can be water or an aqueous ethanol solution having a lower ethanol concentration than the sixth solvent, preferably water or an aqueous ethanol solution having an ethanol concentration of 70 vol% or less (for example, 30 to 70 vol%, preferably 60 vol% or less, for example, 40 to 60 vol%).

The third mixture is a mixture of a hop-derived, xanthohumol-containing component and the fifth solvent. In order to suitably adsorb xanthohumol on PVPP and to prevent adsorption of other components thereon, the fifth solvent is preferably a 55 to 65 vol% aqueous ethanol solution, more preferably a 58 to 63 vol% aqueous ethanol solution. The third mixture may be a solution in which a hop-derived, xanthohumol-containing component is dissolved in the fifth solvent, or may be a suspension of the hop-derived component.

In the production method according to the second embodiment of the present invention, the third mixture may be prepared and contacted with polyvinylpolypyrrolidone. The third mixture may be one that was provided, or may be prepared by the step of preparing the third mixture.

The third mixture may be prepared by any method. In one embodiment, the third mixture preparation step may include extracting a hop-derived, xanthohumol-containing raw material with a seventh solvent containing ethanol. In one embodiment, the third mixture can be prepared by, for example, extracting a hop-derived, xanthohumol-containing raw material with the seventh solvent containing ethanol to prepare a liquid extract (also referred to as a "fourth mixture") containing a hop-derived, xanthohumol-containing component, and optionally adjusting the ethanol concentration in the liquid extract. In one embodiment, the fourth mixture can be used directly as the third mixture.

The hop-derived, xanthohumol-containing raw material and a preferred embodiment thereof are as described above for the production method according to the first embodiment of the present invention.

In one embodiment, when the hop-derived, xanthohumol-containing raw material is extracted with a solvent, preferably, the raw material is washed with a solvent containing water (a raw material washing solvent) before extraction. When the hop-derived raw material is washed with a solvent containing water before extraction, the resulting xanthohumol-containing composition has a higher xanthohumol content in solids. In one embodiment, preferably, the hop-derived, xanthohumol-containing raw material is washed with a solvent containing water, and the hop-derived, xanthohumol-containing raw material is then extracted with the seventh solvent containing a water-miscible solvent to prepare a liquid extract containing a hop-derived, xanthohumol-containing component (the fourth mixture) so as to prepare the third mixture.

The method of washing the hop-derived raw material, the raw material washing solvent, preferred embodiments thereof, and the like are as described above for the production method according to the first embodiment of the present invention. When the raw material washing solvent contains a water-miscible solvent, the water-miscible solvent concentration is preferably lower than the water-miscible solvent concentration in the seventh solvent used for extraction. The water-miscible solvent and a preferred embodiment thereof are as described above, and ethanol is preferred. In one embodiment, when the raw material washing solvent contains a water-miscible solvent, the water-miscible solvent concentration is preferably 30 vol% or less. When the water-miscible solvent concentration in the raw material washing solvent is 30 vol% or less, the resulting xanthohumol-containing composition has a higher xanthohumol content in solids. In order to prevent the loss of xanthohumol from the raw material, the water-miscible solvent concentration in the raw material washing solvent is preferably 30 vol% or less. After washing, the hop-derived raw material may be separated from the solvent and subjected to extraction with the seventh solvent.

In order to efficiently extract xanthohumol from the hop-derived raw material, the seventh solvent used for extraction is preferably a 35 to 90 vol% aqueous ethanol solution, more preferably a 40 to 70 vol% aqueous ethanol solution, still more preferably a 50 to 70 vol% aqueous ethanol solution. The extraction method, a preferred embodiment, and the like are as described above for the production method according to the first embodiment of the present invention.

Preferably, the extraction is followed by removal of insoluble, hop-derived raw material residue from the liquid extract. The method of removing insoluble residue from the liquid extract is not limited, and any of the known separation means described above can be used. The thus-obtained liquid extract of the hop-derived raw material is collected as a mixture containing a hop-derived, xanthohumol-containing component (the fourth mixture). The water concentration and the ethanol concentration in the liquid extract (the fourth mixture) obtained above is optionally adjusted, whereby the third mixture containing a 50 to 70 vol% aqueous ethanol solution can be prepared. In one embodiment, the solvent is partially or entirely removed from the fourth mixture to prepare the third mixture.

In an example of a preferred embodiment, the hop-derived, xanthohumol-containing raw material can be extracted with a 50 to 70 vol% aqueous ethanol solution, and the resulting liquid extract (the fourth mixture) can be used as the third mixture.

In one embodiment, the third mixture can also be prepared by using hop extract or the like and mixing a hop-derived, xanthohumol-containing component with a 50 to 70 vol% aqueous ethanol solution. In the present invention, a xanthohumol-containing hop extract, its treated product, or the like may be mixed with water and ethanol to prepare the third mixture. The xanthohumol-containing hop extract and its treated product can be the above-described xanthohumol-containing hop extract, its treated product, or the like, which can be used for preparing the first mixture. In one embodiment, a concentrate or dried product of the liquid extract of the hop-derived raw material obtained by partially or entirely removing the solvent from the fourth mixture is mixed with water and ethanol, whereby the third mixture can be prepared. The third mixture can also be prepared by using a xanthohumol-containing eluate obtained by the elution step (described later) and optionally adjusting the water concentration and the ethanol concentration. A xanthohumol-containing composition obtained by removing the solvent from the eluate can also be used as a hop-derived, xanthohumol-containing component. It is possible to further increase the xanthohumol content by performing the present invention by using a xanthohumol-containing composition obtained by the production method of the present invention.

The hop-derived component content in the third mixture is not limited. For example, the content may be 3 wt% or less in the mixture. The third mixture may contain a substance other than the hop-derived component and the aqueous ethanol solution, as long as the effect of the present invention is not impaired.

In the production method according to the second embodiment of the present invention, in the adsorption step, the third mixture is contacted with polyvinylpolypyrrolidone to adsorb xanthohumol in the third mixture on polyvinylpolypyrrolidone. Polyvinylpolypyrrolidone is as described above. The method of contacting the third mixture with polyvinylpolypyrrolidone can be the same as the method in the adsorption step of the production method according to the first embodiment. In the adsorption step, a preferred embodiment of the contact time between PVPP and the third mixture is as described for the adsorption step of the production method according to the first embodiment. The amount of PVPP can be the same as that in the production method according to the first embodiment. The amount (in weight) of PVPP is preferably 5 to 150 times, more preferably 5 to 75 times, still more preferably 6 to 35 times, particularly preferably 7 to 25 times the amount of xanthohumol in the third mixture. After the third mixture is contacted with PVPP, preferably, PVPP containing xanthohumol adsorbed thereon is separated from the mixture containing the solvent. Any of the known methods described above may be used to separate PVPP.

In the elution step, xanthohumol is eluted from polyvinylpolypyrrolidone that underwent the adsorption step with the sixth solvent, whereby a xanthohumol-containing eluate is obtained. The sixth solvent is ethanol or an aqueous ethanol solution having a higher ethanol concentration than the fifth solvent. The elution step can be performed by contacting the above-described PVPP with the sixth solvent. The method of contacting the sixth solvent with PVPP can be the same as the method in the elution step of the production method according to the first embodiment. A preferred range of the amount of the sixth solvent may be the same as the preferred range of the third solvent in the production method according to the first embodiment.

The sixth solvent is preferably ethanol or a 70 or more vol% aqueous ethanol solution, more preferably a 70 or more vol% aqueous ethanol solution, still more preferably a 70 to 99 vol% aqueous ethanol solution. In order to improve the yield of xanthohumol, the ethanol concentration in the aqueous ethanol solution used as the sixth solvent is more preferably 70 to 95 vol%, particularly preferably 70 to 92 vol%, most preferably 70 to 90 vol%.

In the elution step, a preferred embodiment of the contact time between PVPP and the solvent used for elution (the sixth solvent) is the same as that in the elution step of the production method according to the first embodiment. A xanthohumol-containing eluate can be obtained by the elution step.

Preferably, the elution step is followed by separation of PVPP from the eluate. The method of separating the eluate from PVPP is not limited, and a known method can be used. For example, when PVPP and the sixth solvent were placed in a container and stirred, PVPP can be separated from the eluate by a method such as filtration or centrifugation. When the elution step is performed with PVPP packed in a column, the sixth solvent is passed through PVPP in the column, whereby the elution of xanthohumol and separation of PVPP from the solvent can be performed.

Described below is an embodiment of the method of obtaining a xanthohumol-containing eluate by the adsorption step and the elution step, in the production method according to the second embodiment of the present invention.

For example, the third mixture and PVPP are placed in a container and stirred, whereby xanthohumol in the mixture can be adsorbed on PVPP. Then, PVPP containing xanthohumol adsorbed thereon is separated from the solvent. Then, PVPP containing xanthohumol adsorbed thereon and the sixth solvent are placed in a container and stirred, whereby xanthohumol adsorbed on PVPP is eluted. PVPP is separated from the eluate, whereby a xanthohumol-containing eluate can be obtained.

In the above embodiment, after adsorption on PVPP, a column is packed with the collected PVPP and the sixth solvent is passed through the column so as to elute xanthohumol from PVPP and separate and obtain an eluate from PVPP.

In another embodiment to obtain an eluate, a column is packed with PVPP, the third mixture is passed through the column to adsorb xanthohumol on PVPP, and subsequently, the sixth solvent is passed through the column, whereby a xanthohumol-containing eluate can be obtained.

In the production method according to the first embodiment and the production method according to the second embodiment of the present invention (hereinafter also collectively referred to as "the production method of the present invention"), the eluate obtained by the elution step contains the solvent used for elution (the third solvent in the production method according to the first embodiment, or the sixth solvent in the production method according to the second embodiment). In the production method of the present invention, the eluate obtained by the elution step is a composition containing xanthohumol and ethanol, and can be used as a xanthohumol-containing composition in the present invention.

The eluate obtained by the production method of the present invention can be used directly or in the form of a concentrate with the solvent suitably removed, for example, in production of food and beverages, pharmaceutical products, raw materials thereof, and the like. The eluate has a high xanthohumol content in solids, and a composition having a high xanthohumol content can be obtained by removing the solvent from the eluate. In one embodiment, preferably, the production method of the present invention includes a step of removing the solvent from the xanthohumol-containing eluate (solvent removal step).

The method of removing the solvent from the eluate is not limited, and any known method can be used. For example, a method such as reduced pressure drying, vacuum drying, or freeze drying can be used. Alternatively, the eluate may be adjusted to have a water content of 90 vol% or more and a pH of 3.0 or less so as to precipitate and collect xanthohumol, and the collected xanthohumol may be dried by any of the methods described above.

In order to suppress isomerization of xanthohumol, the temperature used in the production method according to the first embodiment and the production method according to the second embodiment of the present invention is preferably 50°C or lower, more preferably 4°C to 30°C, still more preferably 10°C to 30°C.

The xanthohumol-containing composition obtained by the production method of the present invention may be in any form, such as a powder, paste, or liquid. Preferably, it is a powder.

The production method of the present invention can provide a composition having a high xanthohumol content in solids. The xanthohumol content in solids of the xanthohumol-containing composition such as an eluate obtained by the production method of the present invention is preferably 80 wt% or more, more preferably 81 wt% or more, 82 wt% or more, 83 wt% or more, or 84 wt% or more, still more preferably 85 wt% or more, particularly preferably 86 wt% or more, most preferably 90 wt% or more. The xanthohumol content in solids may be 99 wt% or less, or 98 wt% or less. The xanthohumol content in solids of the xanthohumol-containing composition is, for example, preferably 80 to 99 wt%, more preferably 81 to 99 wt%, 82 to 99 wt%, 83 to 99 wt%, or 84 to 99 wt%, still more preferably 85 to 99 wt%, particularly preferably 86 to 99 wt%, most preferably 90 to 99 wt%.

In one embodiment of the present invention, the solvent removal step, if performed, results in a composition having a high xanthohumol content, for example, a xanthohumol-containing composition having a xanthohumol content of 80 wt% or more. The xanthohumol content of the xanthohumol-containing composition obtained by the production method of the present invention is preferably 80 wt% or more, more preferably 81 wt% or more, 82 wt% or more, 83 wt% or more, or 84 wt% or more, still more preferably 85 wt% or more, particularly preferably 86 wt% or more, most preferably 90 wt% or more. The xanthohumol content of the composition may be 99 wt% or less, or 98 wt% or less. The xanthohumol content of the xanthohumol-containing composition is, for example, preferably 80 to 99 wt%, more preferably 81 to 99 wt%, 82 to 99 wt%, 83 to 99 wt%, or 84 to 99 wt%, still more preferably 85 to 99 wt%, particularly preferably 86 to 99 wt%, most preferably 90 to 99 wt%. The production method of the present invention can be used as a method of producing a xanthohumol-containing composition having a xanthohumol content in the above ranges. The xanthohumol content can be measured by high performance liquid chromatography (HPLC).

In one embodiment, the production method of the present invention can produce a composition having a high xanthohumol content in which the xanthohumol content is in the above ranges. The production method of the present invention can produce a xanthohumol-containing composition in which the xanthohumol content is in the above ranges, without performing a recrystallization step. A composition obtained in a preferred embodiment of the production method of the present invention is a composition having a high xanthohumol content (a xanthohumol-rich composition), and can be used as high-purity xanthohumol. The production method of the present invention can be used as a method of purifying xanthohumol from a hop-derived, xanthohumol-containing component or a method of producing high-purity xanthohumol. The xanthohumol-containing composition obtained by the present invention can be widely used in applications such as production of food and beverages such as health food, pharmaceutical products, and raw materials thereof.

### EXAMPLES

Hereinafter, the present invention is described in further detail with reference to examples, but the scope of the present invention is not limited thereto.

The xanthohumol content of a raw material used in the examples and the comparative examples and the xanthohumol content of each resulting powder were measured by high performance liquid chromatography (HPLC).

### <HPLC system>

High pressure gradient pump: LC-30AD (two units) (Shimadzu Corporation)
Autosampler: SIL-30AC (Shimadzu Corporation)
Column oven: CTO-20AC (Shimadzu Corporation)
Photodiode array detector: SPD-M20A (Shimadzu Corporation)
<HPLC measurement conditions>
Column: YMC-Triart C18, 2.1 × 150 mm (YMC Co., Ltd.)
Mobile phase: solvent A: 0.1% formic acid-distilled water;
solvent B: 0.1% formic acid in acetonitrile
Gradient conditions: The concentration of solvent B is
retained at 50% from 0 to 6 min; increased to 100% from 6 to 8 min for linear gradient elution; returned to 50% from 15 to 16 min; and retained at equilibrium from 16 to 20 min.
Column temperature: 40°C
Flow rate: 0.2 mL/min
Injection volume: 2 µL
Detection wavelength: UV 367 nm

A sample (powder) was dissolved in a 20 vol% aqueous acetonitrile solution to a concentration of 0.02 mg/mL, whereby a sample for HPLC measurement was prepared. The sample was analyzed under the above conditions to measure the xanthohumol content.

Residue of hops used below treated with supercritical carbon dioxide (supercritical CO₂) is Spent Hop (trade name) available from Hopsteiner. Polyvinylpolypyrrolidone (PVPP) was Divergan (trade name) available from BASF.

In the examples and the comparative examples, the operation was performed at room temperature (25°C).

### <Example 1>

### (Extraction)

Residue (33 g) of hop treated with supercritical CO₂ was subjected to stirring extraction in a 50 vol% aqueous ethanol solution (330 mL) for one hour. After extraction, the filtrate was collected by filtration through filter paper, whereby xanthohumol-containing liquid hop extract was obtained.

### (PVPP adsorption)

To the resulting liquid hop extract was added water to adjust the ethanol concentration to 30 vol%. To the adjusted liquid extract was added PVPP (2 g), followed by stirring for one hour to adsorb the target component thereon. Subsequently, the PVPP was collected by filtration through filter paper.

### (Washing)

To the collected PVPP was added a 50 vol% aqueous ethanol solution whose volume was five times that of PVPP for contact for 30 minutes. Subsequently, the PVPP was collected by filtration through filter paper.

### (Elution)

To the collected PVPP was added a 90 vol% aqueous ethanol solution whose volume was 10 times that of PVPP for contact for 30 minutes to elute adsorbed components. Subsequently, a xanthohumol-rich solution (eluate 1) was separated from the PVPP by filtration through filter paper. To the collected PVPP was again added a 90 vol% aqueous ethanol solution whose volume was 10 times that of PVPP for contact for 30 minutes to elute adsorbed components, whereby a xanthohumol-rich solution (eluate 2) was obtained. The resulting eluates were concentrated under reduced pressure, whereby a yellow powder was obtained. HPLC analysis showed that the powder (0.187 g) obtained from the eluate 1 contained 80 wt% xanthohumol. The analysis also showed that the powder (0.058 g) obtained from the eluate 2 contained 97 wt% xanthohumol. The yield of xanthohumol was 63%. When the powder obtained from the eluate 1 and the powder obtained from the eluate 2 were combined, the xanthohumol content of the powder obtained in Example 1 was 84 wt%.

The yield of xanthohumol is based on the weight of xanthohumol in the used raw material taken as 100%.

### <Example 2>

### (Extraction)

Residue (50 g) of hop treated with supercritical CO₂ was subjected to stirring extraction in a 50 vol% aqueous ethanol solution (400 mL) for one hour. The resulting extract was filtered through a Buchner funnel, and the residue was washed with a 50 vol% aqueous ethanol solution (150 mL). The resulting filtrate was collected and liquid hop extract (472.5 mL) was obtained.

### (PVPP adsorption)

To the resulting liquid hop extract was added water to adjust the ethanol concentration to 30 vol%. To the adjusted liquid extract was added PVPP (3 g), followed by stirring for one hour to adsorb the target component thereon. Subsequently, the PVPP was collected by filtration through filter paper.

### (Washing)

To the collected PVPP was added a 50 vol% aqueous ethanol solution whose volume was five times that of PVPP for contact for 30 minutes. Subsequently, a column (trade name: XK column, available from GE Healthcare Bio-Sciences AB) was packed with the PVPP, and a mixture of a 50 vol% aqueous ethanol solution and the PVPP was passed through the column for solid-liquid separation. The filtrate was discarded.

### (Elution)

A 90 vol% aqueous ethanol solution (100 mL) was passed through the column packed with the PVPP at 0.9 mL/min to collect an eluate. The eluate was concentrated under reduced pressure and vacuum dried, whereby a powder (0.392 g) was obtained. HPLC analysis showed that the resulting powder contained 86 wt% xanthohumol. The yield of xanthohumol was 67%.

In the subsequent examples, the liquid was fed to a column at 0.9 mL/min. The column used was the same as the column (trade name: XK column, GE Healthcare Bio-Sciences AB) used in Example 2.

### <Example 3>

### (Extraction)

Residue (33 g) of hop treated with supercritical CO₂ was subjected to stirring extraction in a 50 vol% aqueous ethanol solution (330 mL) for one hour. After extraction, the filtrate was collected by filtration through filter paper, whereby xanthohumol-containing liquid hop extract was obtained.

### (PVPP adsorption)

To the resulting liquid hop extract was added water to adjust the ethanol concentration to 30 vol%. The liquid extract whose ethanol concentration was adjusted to 30 vol% was passed through the column packed with PVPP (2 g) to adsorb the target component thereon. The filtrate was discarded.

### (Washing)

Subsequently, a 50 vol% aqueous ethanol solution (50 mL) was passed through the column to wash the PVPP. The filtrate was discarded.

### (Elution)

Next, a 90 vol% aqueous ethanol solution (150 mL) was passed through the column to collect an eluate. The eluate was concentrated under reduced pressure and vacuum dried, whereby a powder (0.090 g) was obtained. HPLC analysis showed that the resulting powder contained 83 wt% xanthohumol. The yield of xanthohumol was 65%.

### <Example 4>

### (Extraction)

Residue (33 g) of hop treated with supercritical CO₂ was subjected to stirring extraction in a 60 vol% aqueous ethanol solution (330 mL) for one hour. After extraction, xanthohumol-containing liquid hop extract was obtained by filtration through filter paper.

### (PVPP adsorption)

The resulting liquid hop extract was passed through a column packed with PVPP (2 g) to adsorb the target component thereon. The filtrate was discarded.

### (Washing)

Subsequently, a 70 vol% aqueous ethanol solution (50 mL) was passed through the column to wash the PVPP. The filtrate was discarded.

### (Elution)

Next, a 90 vol% aqueous ethanol solution (110 mL) was passed through the column to collect an eluate. The eluate was concentrated under reduced pressure and vacuum dried, whereby a powder (0.088 g) was obtained. HPLC analysis showed that the resulting powder contained 94 wt% xanthohumol. The yield of xanthohumol was 61%.

### <Examples 5, 6, and 7>

In each of these examples, a xanthohumol-containing powder (Example 5: 0.181 g; Example 6: 0.187 g; Example 7: 0.316 g) was obtained by purifying xanthohumol as in Example 2, except that the ethanol concentration in the aqueous ethanol solution was changed in the PVPP washing step. The washing step was performed with a 30 vol% aqueous ethanol solution in Example 5, a 40 vol% aqueous ethanol solution in Example 6, and a 60 vol% aqueous ethanol solution in Example 7.

The xanthohumol-containing powders obtained in Examples 5 to 7 were analyzed by HPLC to determine the xanthohumol content (wt%) (purity) and the yield (%) of xanthohumol. Table 1 shows the results.

**[Table 1]**

| | Concentration of aqueous ethanol solution for washing (vol%) | Xanthohumol content (wt%) | Yield of xanthohumol (%) |
|---|---|---|---|
| Example 5 | 30 | 81 | 31 |
| Example 6 | 40 | 82 | 32 |
| Example 7 | 60 | 80 | 54 |

After washing PVPP with the aqueous ethanol solution, elution was performed with an aqueous ethanol solution having a higher concentration. This resulted in a powder having a xanthohumol content of 80 wt% or more (xanthohumol having a purity of 80% or more).

### <Example 8>

Residue (50 g) of hop treated with supercritical CO₂ was subjected to stirring extraction in a 50 vol% aqueous ethanol solution (500 mL) for one hour. After extraction, xanthohumol-containing liquid hop extract was obtained by filtration through filter paper.

### (PVPP adsorption)

To the resulting liquid hop extract was added water to adjust the ethanol concentration to 30 vol%. To the adjusted liquid extract was added PVPP (3 g), followed by stirring for one hour. Then, the PVPP was collected by filtration through filter paper.

### (Washing)

To the collected PVPP was added a 50 vol% aqueous ethanol solution whose volume was five times that of PVPP for contact for 30 minutes. Subsequently, the PVPP was collected by filtration through filter paper.

### (Elution)

To the collected PVPP was added a 70 vol% aqueous ethanol solution whose volume was 10 times that of PVPP for contact for 30 minutes. Subsequently, a xanthohumol-rich solution (eluate 1) was separated from the PVPP by filtration through filter paper. To the collected PVPP was again added a 90 vol% aqueous ethanol solution whose volume was 10 times that of PVPP for contact for 30 minutes, whereby a xanthohumol-rich solution (eluate 2) was obtained. The resulting eluate (eluate 1 + eluate 2) was concentrated under reduced pressure, followed by vacuum drying. Thus, a xanthohumol-containing powder (0.191 g) was obtained.

### <Examples 9 and 10>

In each of these examples, a xanthohumol-containing powder (Example 9: 0.186 g; Example 10: 0.112 g) was obtained by purifying xanthohumol as in Example 8, except that a 80 vol% aqueous ethanol solution (Example 9) or a 99 vol% aqueous ethanol solution (Example 10) was used instead of the 70 vol% aqueous ethanol solution in the elution step.

The xanthohumol-containing powders obtained in Examples 8 to 10 were analyzed by HPLC to determine the xanthohumol content (wt%) (purity) and the yield (%) of xanthohumol. Table 2 shows the results.

**[Table 2]**

| | Concentration of aqueous ethanol solution for elution (vol%) | Xanthohumol content (wt%) | Yield of xanthohumol (%) |
|---|---|---|---|
| Example 8 | 70 | 83 | 49 |
| Example 9 | 80 | 84 | 48 |
| Example 10 | 99 | 81 | 28 |

### <Comparative Example 1>

A xanthohumol-containing powder (0.089 g) was obtained by purifying xanthohumol as in Example 2, except that the ethanol concentration in the aqueous ethanol solution used in the washing step was changed to 90 vol% and the ethanol concentration in the aqueous ethanol solution used in the elution step was changed to 50 vol%. HPLC analysis showed that the resulting powder contained 61 wt% xanthohumol.

In each of Examples 1 to 10, xanthohumol was adsorbed on PVPP, followed by washing with an aqueous ethanol solution, elution with a solvent having a higher ethanol concentration than the aqueous ethanol solution used for washing, and removal of the solvent from the eluate, whereby a powder having a xanthohumol content of 80 wt% or more (xanthohumol having a purity of 80% or more) was obtained.

### <Example 11>

### (Extraction)

Residue (33 g) of hop treated with supercritical CO₂ was subjected to stirring extraction in a 60 vol% aqueous ethanol solution (330 mL) for one hour. After extraction, the filtrate was collected by filtration through filter paper, whereby xanthohumol-containing liquid hop extract was obtained.

### (PVPP adsorption)

The resulting liquid hop extract was passed through a column packed with PVPP (5 g) to adsorb the target component thereon. The filtrate was discarded.

### (Elution)

A 90 vol% aqueous ethanol solution (250 mL) was passed through the column to collect an eluate. The eluate was concentrated under reduced pressure and vacuum dried, whereby a powder (0.103 g) was obtained. HPLC analysis showed that the resulting powder contained 86 wt% xanthohumol. The yield of xanthohumol was 58%.

### <Example 12>

Before extraction in obtaining a xanthohumol-containing powder from a hop-derived raw material, the raw material was washed. Specifically, to residue (33 g).of hop treated with supercritical CO₂was added water (330 mL), followed by stirring for one hour. Subsequently, solid-liquid separation was performed by filtration through filter paper, and the residue was collected. To the collected residue was added a 50 vol% aqueous ethanol solution (330 mL), followed by stirring extraction for one hour. After extraction, the filtrate was collected by filtration through filter paper, whereby xanthohumol-containing liquid hop extract was obtained. The subsequent steps were performed as in the PVPP adsorption, washing, and elution in Example 1 to purify xanthohumol, whereby xanthohumol-rich eluates 1 and 2 were obtained. The resulting eluates were concentrated under reduced pressure, whereby yellow powders were obtained. HPLC analysis showed that the powder (0.158 g) obtained from the eluate 1 contained 84 wt% xanthohumol and that the powder (0.67 g) obtained from the eluate 2 contained 91 wt% xanthohumol. The yield of xanthohumol was 61%. When the powder obtained from the eluate 1 and the powder obtained from the eluate 2 were combined, the xanthohumol content of the powder obtained in Example 12 was 90 wt%.

### <Example 13>

Before extraction in obtaining a xanthohumol-containing powder from a hop-derived raw material, the raw material was washed with an aqueous ethanol solution. Specifically, to residue (33 g) of hop treated with supercritical CO₂ was added a 10 vol% aqueous ethanol solution (a raw material washing solvent) (330 mL), followed by stirring for one hour. Subsequently, solid-liquid separation was performed by filtration through filter paper, and the residue was collected. To the collected residue was added water and ethanol to obtain a 50 vol% aqueous ethanol solution (330 mL), followed by stirring extraction for one hour. After extraction, the filtrate was collected by filtration through filter paper, whereby xanthohumol-containing liquid hop extract was obtained. The subsequent steps were performed as in the PVPP adsorption, washing, and elution in Example 1 to purify xanthohumol, whereby xanthohumol-rich eluates 1 and 2 were obtained. The resulting eluates were concentrated under reduced pressure, whereby yellow powders were obtained. Table 3 shows the xanthohumol content in the powder obtained from the eluates 1 and 2.

A xanthohumol-containing yellow powder was obtained by performing the same method described above, except that the raw material washing solvent was a 20 vol% aqueous ethanol solution or a 30 vol% aqueous ethanol solution instead of the 10 vol% aqueous ethanol solution. Table 3 shows the xanthohumol content in each powder.

**[Table 3]**

| Raw material washing solvent | 10 vol% Ethanol | 20 vol% Ethanol | 30 vol% Ethanol |
|---|---|---|---|
| Xanthohumol content (wt%) | 91 | 86 | 87 |

## Claims

1. A method of producing a xanthohumol-containing composition, comprising:
contacting a first mixture of a hop-derived, xanthohumol-containing component and a first solvent containing water and a water-miscible solvent with polyvinylpolypyrrolidone to adsorb xanthohumol on the polyvinylpolypyrrolidone;
washing the polyvinylpolypyrrolidone containing xanthohumol adsorbed thereon with a second solvent; and
eluting xanthohumol from the polyvinylpolypyrrolidone with a third solvent to obtain a xanthohumol-containing eluate,
wherein the second solvent is an aqueous ethanol solution, and the third solvent is ethanol or an aqueous ethanol solution having a higher concentration than the second solvent.

2. The production method according to claim 1, wherein the second solvent is a 30 to 70 vol% aqueous ethanol solution.

3. The production method according to claim 1 or 2,
wherein the third solvent is a 70 or more vol% aqueous ethanol solution.

4. The production method according to any one of claims 1 to 3,
wherein the first solvent contains 30 to 80 vol% water and a 20 to 70 vol% water-miscible solvent.

5. The production method according to any one of claims 1 to 4,
wherein the water-miscible solvent is ethanol.

6. A method of producing a xanthohumol-containing composition, comprising:
contacting a third mixture of a hop-derived, xanthohumol-containing component and a fifth solvent with polyvinylpolypyrrolidone to adsorb xanthohumol on the polyvinylpolypyrrolidone; and
eluting xanthohumol from the polyvinylpolypyrrolidone containing xanthohumol adsorbed thereon with a sixth solvent to obtain a xanthohumol-containing eluate,
wherein the fifth solvent is a 50 to 70 vol% aqueous ethanol solution, and the sixth solvent is ethanol or an aqueous ethanol solution having a higher concentration than the fifth solvent.

7. The production method according to claim 6,
wherein the sixth solvent is a 70 or more vol% aqueous ethanol solution.

8. The production method according to any one of claims 1 to 7, further comprising a step of removing the solvent from the xanthohumol-containing eluate.

9. The production method according to any one of claims 1 to 8,
which is a method of producing a composition having a xanthohumol content of 80 to 99 wt%.
